# EUROPEAN PATENT SPECIFICATION

(11) **EP 1 708 726 B1**
(45) Date of publication and mention of the grant of the patent: **15.10.2008**
(21) Application number: 05700833.6
(22) Date of filing: 12.01.2005
(51) Int. Cl.: A61K 36/82

(54) **FORMULATION FOR TREATING OBESITY AND ASSOCIATED METABOLIC SYNDROME**
FORMULIERUNG ZUR BEHANDLUNG VON FETTLEIBIGKEIT UND ASSOZIIERTEM STOFFWECHSELSYNDROM
FORMULATION DESTINEE AU TRAITEMENT DE L'OBESITE ET DU SYNDROME METABOLIQUE ASSOCIE

(30) Priority: 15.01.2004 PL 36441104
(43) Date of publication of application: 11.10.2006
(73) Proprietor: Scandinavian Clinical Nutrition I Sverige AB, 114 56 Stockholm (SE)
(72) Inventor: KROTKIEWSKI, Marcin, S-436 39 Askim (SE)
(74) Representative: Örtenblad, Bertil Tore
(86) International application number: PCT/EP2005/000206
(87) International publication number: WO 2005/067952

(56) References cited:
- FR-A- 2 712 191
- US-A- 4 598 089
- US-A- 5 804 596
- DULLOO A G ET AL: "Efficacy of a green tea extract rich in catechin polyphenols and caffeine in increasing 24-h energy expenditure and fat oxidation in humans" AMERICAN JOURNAL OF CLINICAL NUTRITION, BETHESDA,MD, US, vol. 70, no. 6, December 1999 (1999-12), pages 1040-1045, XP001154724 ISSN: 0002-9165 cited in the application
- ANONYMOUS: "Les suppléments nutritionnels de la minceur"[Online] 1 May 2004 (2004-05-01), page 1-5, XP002330877 Retrieved from the Internet: URL:http://www.nutranews.org/fra/index.php ?articleid=4024> [retrieved on 2005-06-07]
- ANONYMOUS: "Fat burners overview"[Online] page 1-8, XP002330878 Retrieved from the Internet: URL:http://www.realsolutionsmag.com/fat-bu rners/fat-burners.htlm> [retrieved on 2005-06-07]
- ANONYMOUS: "Evoplex TM"[Online] page 1-5, XP002330879 Retrieved from the Internet: URL:http://www.befit.ca/evoplex.html> [retrieved on 2005-06-07]
- ANONYMOUS: "FatBlaster MAX"[Online] page 1-2, XP002330880 Retrieved from the Internet: URL:http://www.discountnaturalhealth.com/p rod1540.htm> [retrieved on 2005-06-07]

## Description

The invention relates to a formulation for treating obesity and associated metabolic syndrome

Obesity is increasing at an alarming rate in most countries. It is now well recognized that this development dramatically increases the incidence of type 2 diabetes and to some extent cardiovascular disease, hypertension and cancer - known as metabolic syndrome contributing to the reduction of the life expectancy by several years. During the second half of 20^{th} century the rate of obesity has increased 5-10 fold and included countries of Far East and Asia suffering previously from undernutrition as the biggest problem. Epidemic of obesity is progressing very rapidly and constitutes the biggest health problem of the modem world

Many of the available methods of treating obesity cause only a temporary loss of weight and after the termination of dietary treatment, the patient's are facing a rapid weight regain. Slimming therapies involve primarily increased activity - physical exercises and reduced intake of calories. Discontinuation of the diet is associated with regain of body weight and so called jo-jo effect. Increasing difficulties along with continuation of weight reducing programs based on calorie restriction and the rapid weight regain are considered to be caused by the decrease of metabolic rate and thermogenesis. The decrease depends on the adaptation of the body to the changed energy balance and decreased food intake. Adaptation to low calorie intake includes among other decreased activity of the sympathetic nervous system and changes in the metabolism of thyroid hormones-towards production of the less active forms of the these hormones. Decreased activity of adrenergic nervous system and changed concentration of the active form of thyroid hormones lead to decreased mobilization and oxidation of fatty acids and the decreased activity /expression of uncoupling proteins UCP 1-3. The decrease of metabolic rate is one of the main reasons to the low rate of success of obesity treatment with more than 90% of the slimming patients returning to the start weight.

In view of these known facts it is not surprising to find that other methods of treating obesity involve efforts to increase the metabolic rate. Calories stored in the body, mainly in a form of fat, are burned quicker due to administration of various thermogenetic drugs, which results in loss of body weight. However, those therapies often have negative side effects and, again even here after discontinuation of the therapy patient's body weight goes back to the start values before treatment.

Different types of vegetable extracts are often used as adjuncts in the dietary treatment of obesity. One of the agents recently used is Green tea extract. (Green tea, *Camellia sinensis*) comprises large amount of catechin polyphenols, mainly epigallocatechin gallate- EGCG. Epigallocatechin gallate is a very strong antioxidant, it also reduces the appetite with leads to decrease of food intake (see Am. J. Clin. Nutr 2000, 72, pages 1232-1234). Green tea extracts have been described to inhibit carbohydrates and lipids digestion and exhibit strong antinflammatory activity. Thermogenetic effect of Green tea activity has been described in Am. J. Chin. Nutr. 1999, 70, pages 1040-1045. Authors were reporting that extract rich in polyphenols and caffeine is effectively and significantly increasing energy expenditure and fat oxidation.

Another very popular therapeutic plant is a herb growing in South America called Yerba Maté (Paraguay, *Ilex paraguariensis*), containing triterpenes, caffeine and caffeine-like compounds. In whole America *Ilex paraguariensis* is used in a form of teas and infusions even more often than coffee. In Germany, Yerba Maté leaves are used in an aqueous infusion dosage form in treatment of urinary tract and headaches. Formulations comprising Yerba Maté leaves are also used in treating mental and physical fatigue and are often included in weight loss programs as oral and topical supplementary agents. Pharmaceutical activity of Yerba Maté is ascribed to caffeine, caffeoylquinic acids and caffeine-like polyphenols.

Similar weight-reduction activity is also exhibited by another plant-Guarana (*Paulinia cupana, P. sorbolis*) which contains large amounts of caffeine and other polyphenoles-chlorogenic acids (CGA). According to Hurel, J.P. 1993 (FR 2.712.191 A1), caffeine included in Guarana extracts is the primary agent responsible for body weight decrease. However, use of Yerba Maté or Guarana as a single agent will result only in a momentary effect and after finishing the treatment immediate increase of body weight is observed.

US patent document no. 5.804.596 discloses the activity of *Coleus Forskholii* root extract (ForsLean®). This extract contains active agent - forskolin (diterpene forskolin). Biological mechanism of diterpene forskolin activity is widely described in medical literature and many clinical evaluations have been performed. Those evaluations relate to different activities of diterpene forskolin e.g. broncholytic (for treating bronchial asthma), relaxation of the arteries (for treating hypertension and cardiovascular system disorders), treating glaucoma and impotence. Studies conducted on rats showed, that low doses of diterpene forskolin are not effective. Only use of very high doses resulted in distinct body weight reduction. However, in case of humans such high doses would cause hypotensive effects and harmful high inotropic activity on heart muscle.

Pharmacological activity of birch leaves (*Betula Alba, betulae folium, Batula pendula Roth*) was not a subject of such extensive study as in case of herbs described above but it was widely used in Europe during centuries. Birch leaves contain approx. 2-3 % of flavonoid glycosides as well as triterpen alcohols and esters, previously regarded as saponines. Fresh birch leaves comprise also approx. 0.5 % of ascorbic acid. Aqueous extract of birch leaves is known as mild diuretic agent and is used to irrigate urinary tract, to remove sand and to prevent inflammation of urinary tract. It was also used orally.

The human obesity can be also treated with Orlistat® - ([2S-[2a(R*),3b]]N-formyl-L-leucine 1-[(3-hexyl-4-oxo-2-oxetanyl)methyl]dodecyl ester) being sold under the trademark Xenical® (US patent no. 4.598.089). Daily dose of this drug is three capsules containing 120 mg of Orlistat®. Inhibition of lipase activity evoked by Orlistat means that 30% of consumed fat goes through the digestive tract without decomposition and is not absorbed. However, said drug exhibit some side effects such as: fatigue, headaches, stomachaches, oily diarrheas, gases and flatulence. It is not suitable for children and should not be used by pregnant and breast-feeding women.

The object of the invention is to provide a composition for treating human obesity, which would be effective in accelerating weight loss and which would be characterized by the lack of major side effects and would help to maintain the lower body weight, obtained during slimming.

Surprisingly, it has been found that a certain combination of different herbs and extracts of plant origin acts more effective than each of the components alone. The components are acting in concert (synergistically) strengthening together the two main effects of the mixture i.e. - termogenetic effects (increased metabolic rate) and the decrease of the absorption of fat.

According to the invention, formulation for treating obesity and associated metabolic syndrome, comprising a combination of selected vegetable extracts, consists of:
a) 20-90% wt. of Green tea extract, containing more than 70 % of catechines, preferably containing Epigallocatehin galate EGCG
b) 2-30 % wt. of *Coleus forskholii* extract, containing at least 10 % of diterpene forskolin
c) 5-58 % wt. of Yerba Maté extract, containing 2-4 % of caffeine and caffeoylquinic acids (CGA)
d) 7.5-45 % wt. of *Betula alba* extract containing at most 3% of flavonides.

In other embodiment, Guarana extract is used instead of Yerba Maté extract. The following composition of the formulation was used:
a) 20-80% wt. of Green tea extract, containing more than 70 % of catechines, preferably containing Epigallocatehin galate EGCG,
b) 2-30 % wt. of *Coleus forskholii* extract, containing at least 10 % of diterpene forskolin,
c) 5-50 % wt. of Guarana extract, containing more than 8 % of caffeine and caffeine-like polyphenols (chlorogenic acids-CGA),
d) 7.5-45 % wt. of *Betula alba* extract containing at most 3% of flavonides.

Formulation of the invention may further comprise an effective amount of vegetable extract of white kidney beans (*Phaseolus Vulgaris*)*.*

Preferably, Green tea extract is an extract obtained by water and/or ethyl acetate + water extraction in low temperature under reduced pressure.

In other preferred embodiment, Green tea extract is an extract obtained by alcohol extraction or extraction conducted in the presence of fat solvents, for example selected from a group comprising: methanol-chloroform mixture, alcohol ethers and detergents, in low temperature under reduced pressure.

In one embodiment, Green tea extract comprises at least 30 % of EGCG. In another embodiment, Green tea extract comprises at least 50 % of EGCG, and in another one Green tea extract comprises at least 80 % of EGCG

Depending of the dosage forms, formulation of the invention may comprise non-active exicipients or fillers selected from a group consisting of: silicon dioxide, magnesium stearate, laurylsulphate, other surfactants for example selected from a group consisting of: sodium carboxymethylcellulose, hydroxypropylmethyl cellulose and microcrystalline cellulose, anty-caking agents such as dicalcium phosphate; and materials forming the shell of the capsule.

According to in vitro and in vivo animal and humans studies, the application of formulation of the invention is effectively accelerating weight loss.

One of the most important advantages of the formulation of the invention apart from the acceleration of weight loss is the prevention of weight regain after the termination of slimming cure. Preparation is also showing hypo-lipidemic and antihypertensive effect.

Formulation of the invention may be used in a form of infusions, teas, capsules, tablets, chewing gums and powders which can be dissolved in water.

Daily dose of the formulation in case of humans is equal to:
250-2000 mg of Green tea extract,
20-300 mg of *Betula alba* extract,
30-400 mg of Yerba Maté or
20-350 mg of Guarana,
30-600 mg of *Coleus forskholii.*

In case of addition of white kidney beans extract in the formulation, the daily dose for human would be 500-4000 mg of white kidney bean extract.

The invention has been illustrated by examples and drawings on which:
Fig.1 shows a comparison between lipase inhibitory activity of the formulation of the invention and Xenical® formulation.
Fig. 2A shows comparisons of the slowing down of the increases of body weight of growing rats after 12 weeks of diet supplemented with: either the formulation of the invention or pure extracts of Yerba mate, Guarana, *Coleus forskholii*, *Betula alba* and Green tea (EGCG).
Fig 2B shows comparison of the mean changes of rats body weight after 12 weeks of diet supplemented with: either the formulation of the invention or pure extracts of Yerba mate, Guarana, *Coleus forskholii, Betula alba* and Green tea (EGCG).
Fig. 3 shows comparisons between mean food intake in rats in groups treated with: the formulation of the invention and pure extracts of Yerba mate, Guarana, *Coleus forskholii, Betula alba,* Green tea (EGCG) after 12 weeks.
Fig.4 shows the effect of the formulation of the invention on resting metabolic rate on 12 healthy volunteers.
Fig.5 shows the change of body weight (in %) during consecutive 14 weeks on patients following a low calorie diet supplemented with the invention in comparison with patients following same low calorie diet and supplemented with placebo (study followed the rules of good clinical practice and was performed at university hospital).
Fig.6 shows the decrease of body fat weight (in kg) after 14 weeks of low calorie diet supplemented with the formulation of the invention or placebo.
Fig.7 shows the decrease of LDL plasma cholesterol concentration (in mg/dl) after 14 weeks of low calorie diet supplemented with the formulation of the invention or placebo.
Fig.8 shows the decrease of total plasma cholesterol concentration (in mg/dl) after 14 weeks of low calorie diet supplemented with the formulation of the invention or placebo.
Fig.9 shows the decrease of total plasma triglycerides concentration (in mg/dl) after 14 weeks of low calorie diet supplemented with the formulation of the invention or placebo.

### Example 1 Capsule composition

The formulation of the invention may be administered in the form of capsules. An example of the capsule composition is presented below.

| | |
|---|---|
| Green tea extract | 325 mg, |
| *Betula alba* | 30 mg, |
| Yerba Maté | 30 mg, |
| *Coleus forskholii* | 10 mg, |
| Total weight of active agents | 395 mg, |
| Silicon dioxide | 12 mg, |
| Magnesium stearate | 5 mg, |
| Lauryl sulphate | 5 mg, |
| Total weight of supplementary components | 22 mg, |
| Capsule shell | 95 mg, |
| Total weight of the capsule | 512 mg. |

### Example 2 Capsule composition

Another example of the capsule composition is presented below.

| | |
|---|---|
| Green tea extract | 325 mg, |
| *Betula alba* | 30 mg, |
| Yerba Maté | 30 mg, |
| *Coleus forskholii* | 10 mg, |
| Total weight of active agents | 395 mg, |
| Silicon dioxide | 12 mg, |
| Magnesium stearate | 5 mg, |
| Total weight of supplementary components | 17 mg, |
| Capsule shell | 95 mg, |
| Total weight of the capsule | 507 mg. |

### Example 3. Inhibiting the lipase activity

The human obesity can be treated by reducing the fats digestion and fat absorption. Fats must be decomposed by lipase before they are absorbed by the organism. Inhibiting lipase activity causes considerable reduction of the fat absorption which decrease calorie intake. Such mechanism illustrates the activity of Roche's anti-obesity formulation Xenical® (Orlistat®).

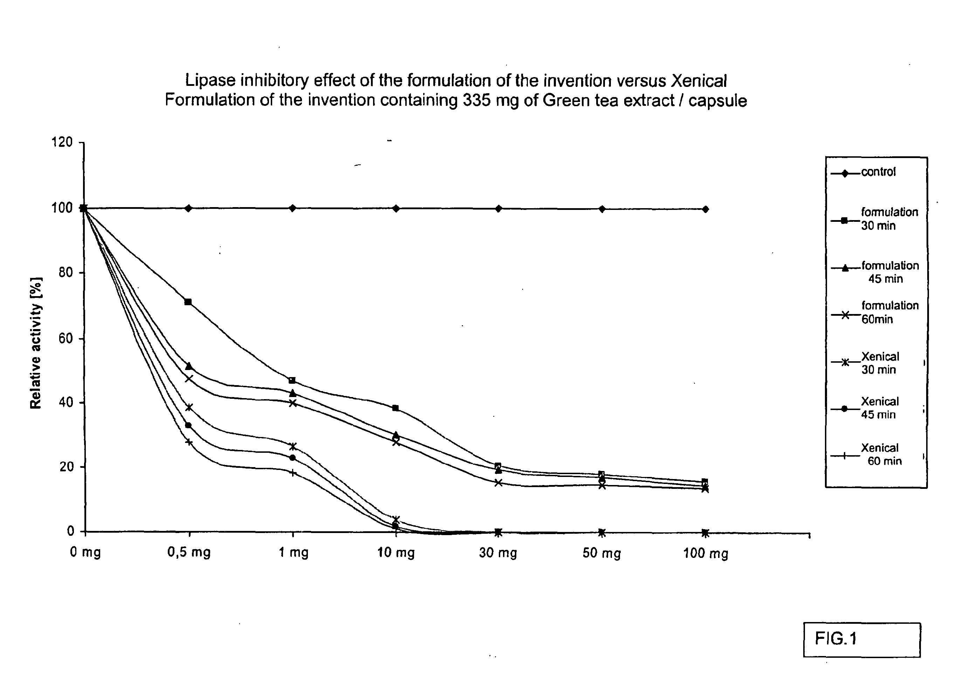

Fig. 1 shows the comparison between in vitro effect of the innovation on the activity of pancreatic lipase and various concentrations of Xenical® (0.5-100 mg) after 30, 45 and 60 minutes in temperature of 37°C. The composition of the formulation of the invention is presented below in the Table 1:

**Table 1**

| Component | % (wt.) |
|---|---|
| Green tea extract | 82.3 |
| *Coleus forskholii* extract | 2.5 |
| Yerba Maté extract | 7.6 |
| *Betula alba* extract | 7.6 |

In the case of the formulation of the invention, the lipase activity was already reduced after 30 minutes. Increase of time period to 45 and 60 minutes resulted in larger reduction of lipase activity in case of lower concentrations (down to 30 mg) of the formulation of the invention. Maximum reduction of lipase activity obtained was equal to approx 80 %. Although, the Xenical® (Orlistat®) inhibits the lipase activity stronger than the formulation of the invention and at concentration of 10 mg after 45 and 60 minutes causes complete inhibition of lipase activity, it leads also to negative side effects described herein.

### Example 4. Effect of the formulation of the invention and its components on reduction of body weight increase in rats.

The study was conducted to prove the influence of formulation of the invention (composition showed in the Table 1) on weight of laboratory rats in comparison to the groups fed with separate ingredients of the formulation.

72 female Wistar rats (initial weight 173-209 g) were breeded in the age of 8 weeks and housed in groups of 6 rats in one cage for 7 days (12h/12h light/dark cycle) in a temperature and humidity controlled conditions. After 7 days of adaptation, healthy rats selected to the experiment were divided into 6 groups, each receiving one of following substances: formulation of the invention, *Coleus forskholii, Betula Alba,* Yerba mate, Green Tea Extract (EGCG) and Guarana. The substances were administered orally (by gavage) in the form of solutions. During the experiment the animals were fed with Ssniff® R (purchased from Spezialdiäten GmbH, Germany)-a certified pellet chow and municipal water. The doses, which were calculated to correspond the human daily doses, are presented in the Table 2.

**Table 2.**

| Substance | Daily dosage [mg] |
|---|---|
| Formulation of the invention | 6.95 |
| *Colleus forskholii* | 0.17 |
| *Betula Alba* | 0.5 |
| Yerba Maté | 0.5 |
| EGCG | 5.33 |
| Guarana | 0.42 |

Body weights were measured and recorded once per week.

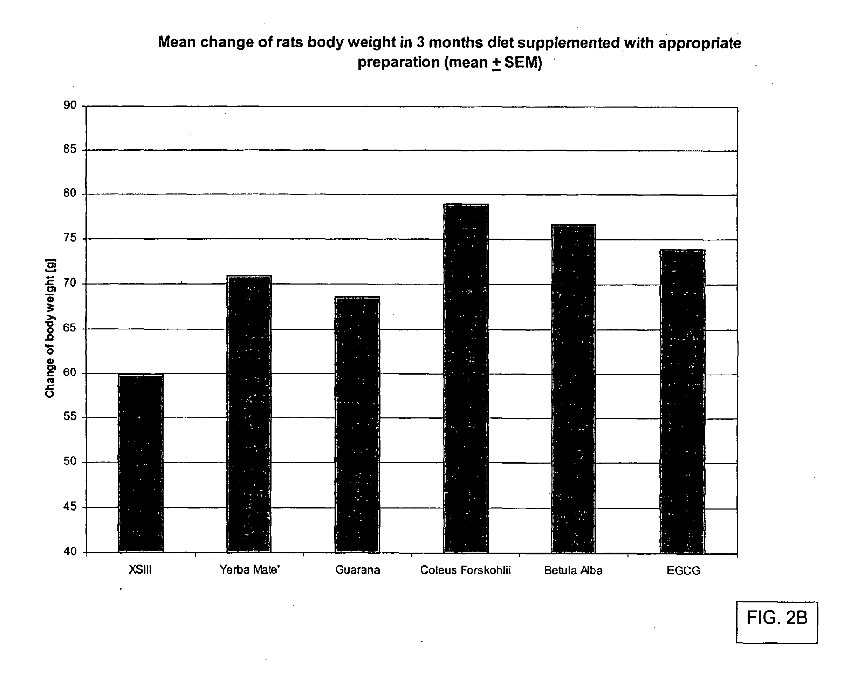

12 weeks of observation of Wistar rats proved that the increase of body weight is on the lowest level in the group treated with the formulation of the invention. Values of the mean body weight increase in tested groups are presented in the Table 3 and fig. 2B.

**Table 3**

| Group treated with | Mean body weight increase [g] |
|---|---|
| Formulation of the invention | 59.8 |
| *Colleus forskholii* | 78.2 |
| *Betula Alba* | 76.7 |
| Yerba Maté | 71.0 |
| EGCG | 73.9 |
| Guarana | 68.7 |

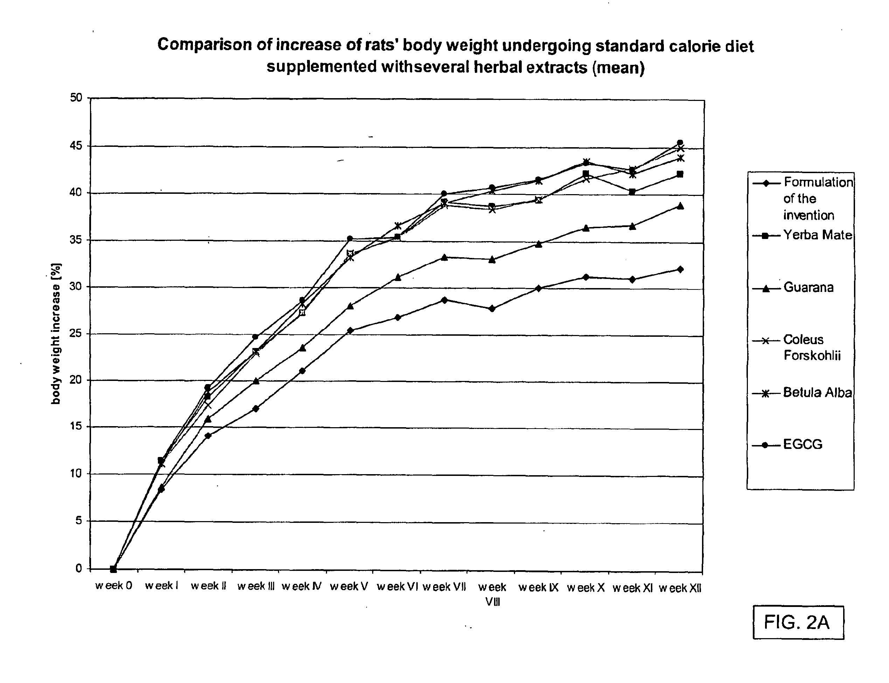

The increase (%) of body weight in rats during whole experiment are also presented on fig. 2A.

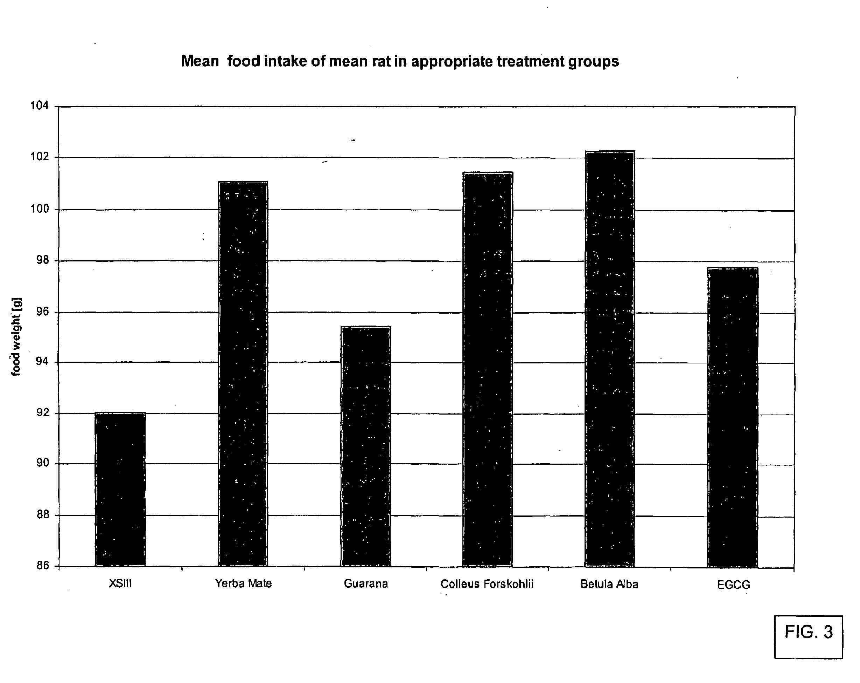

Additionally, the mean amount of pellet-chow eaten by rats in appropriate group was measured. The results are presented on the fig. 3. It is clear that the mean food intake is lowest in the group treated with the formulation of the invention.

The experiment proved that combination of the natural substances present in the formulation of the invention is effective in reducing the body weight in rat, and acts much stronger than its separate ingredients.

### Example 5. Effect of the formulation of the invention - study conducted on humans.

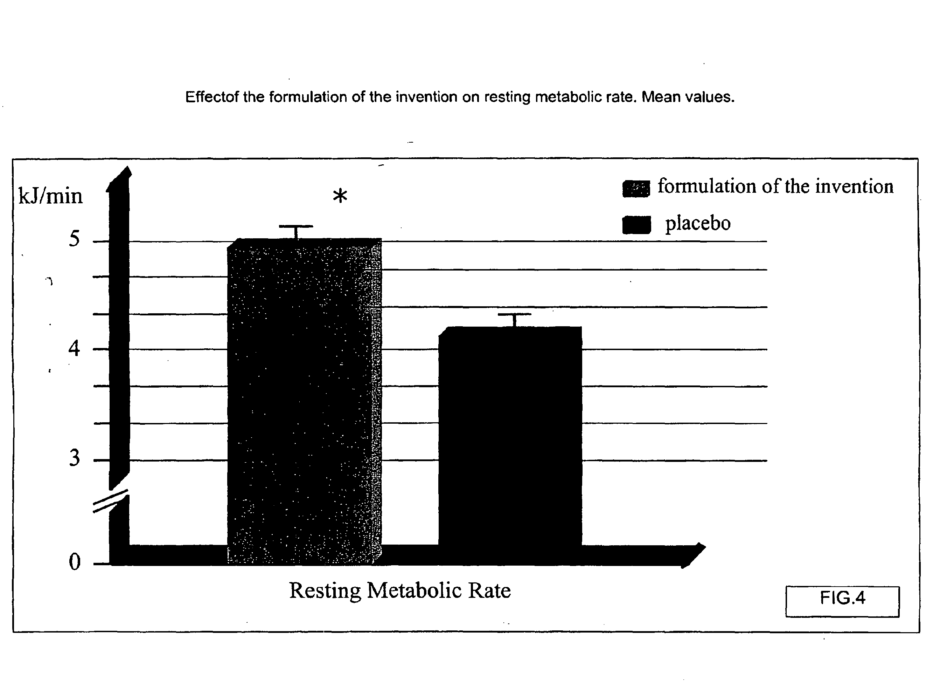

Advantageous properties of the formulation of the invention were confirmed by a study conducted on healthy volunteers. The aim of the study was to check the safety and efficacy of the invention and to examine the influence of the formulation of the invention used as a food supplement on resting metabolic rate. Mean resting metabolic rate increased in the treated group from 4.3 ± 0.2 to 4.8 ± 0.2 kJ/min after 3 days (fig. 4) whereas exercise metabolic rate remained unchanged (not showed).

Another human study-double blind, placebo controlled clinical study- was performed under GCP to examine the decrease of body weight of patients on low calorie diet (1000 kcal/day), body fat weight, increase of non-fat body weight (figure improvement), decrease of plasma LDL cholesterol, total cholesterol and plasma triglycerides concentration. The study lasted 14 weeks. The composition of the formulation of the invention is presented below in the Table 4:

**Table 4**

| Component | % (wt.) |
|---|---|
| Green tea extract | 79.5 |
| *Coleus forskholii* extract | 2.4 |
| Guarana extract | 6.2 |
| *Betula alba* extract | 11.9 |

Influence of formulation of the invention was examined during tests performed on 48 obese patients divided into two groups who completed the study. Both groups were receiving low calorie diet (1000 kcal/day), but diet of only one group was supplemented with the formulation of the invention ("treated group").

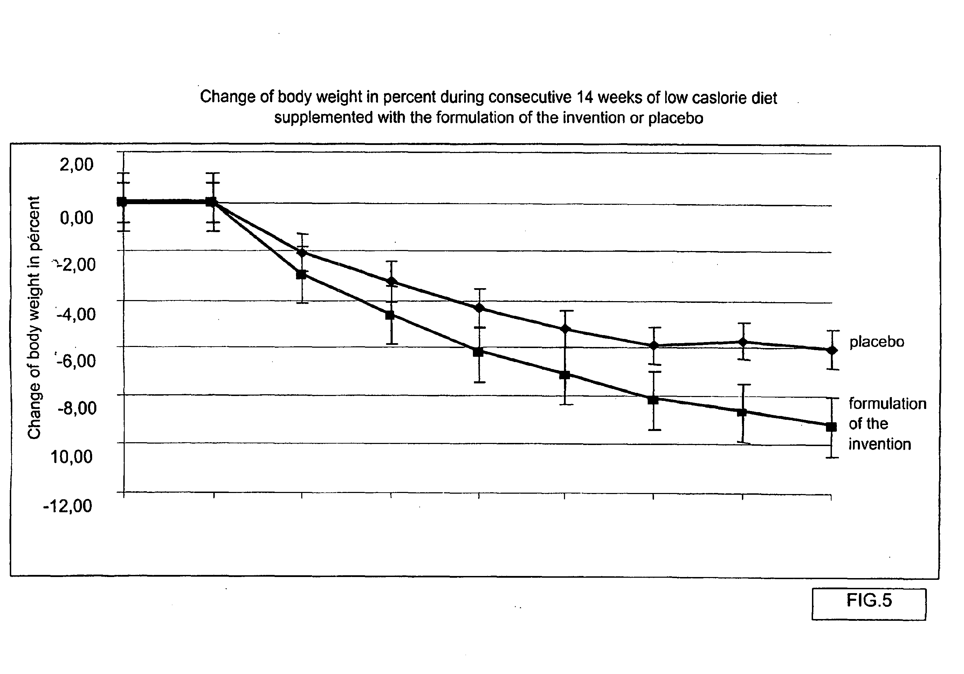

After 14 weeks, body weight decrease was equal 6.06 % in the control group and 9,28 % in the treated group which was receiving the formulation of the invention (fig. 5). Those values expressed in kilograms are equal to 5.54 kg and 8.51 kg respectively. Difference between these two groups was 53.6 %. Differences were found statistically significant.

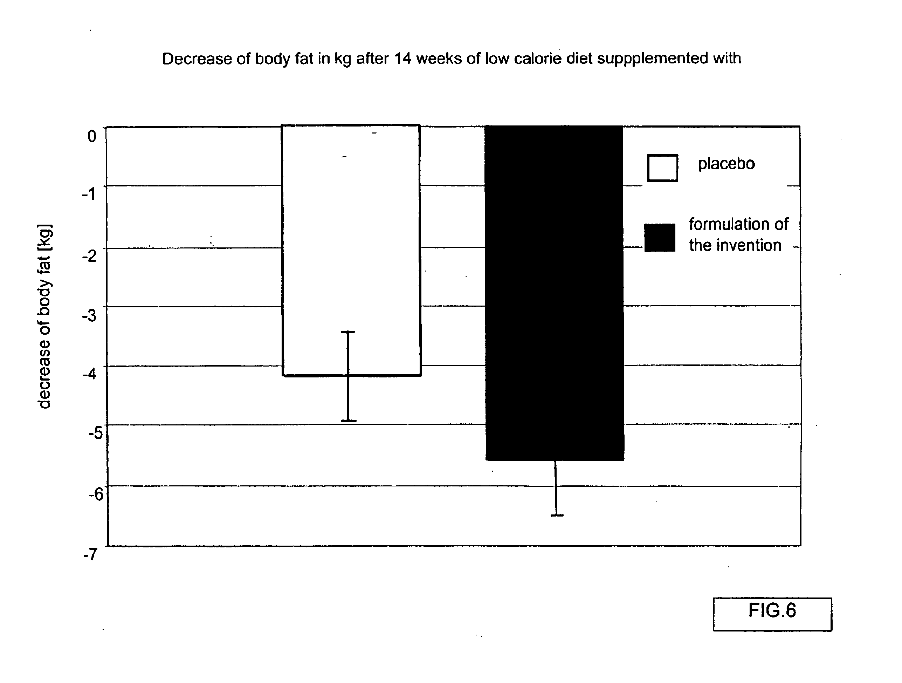

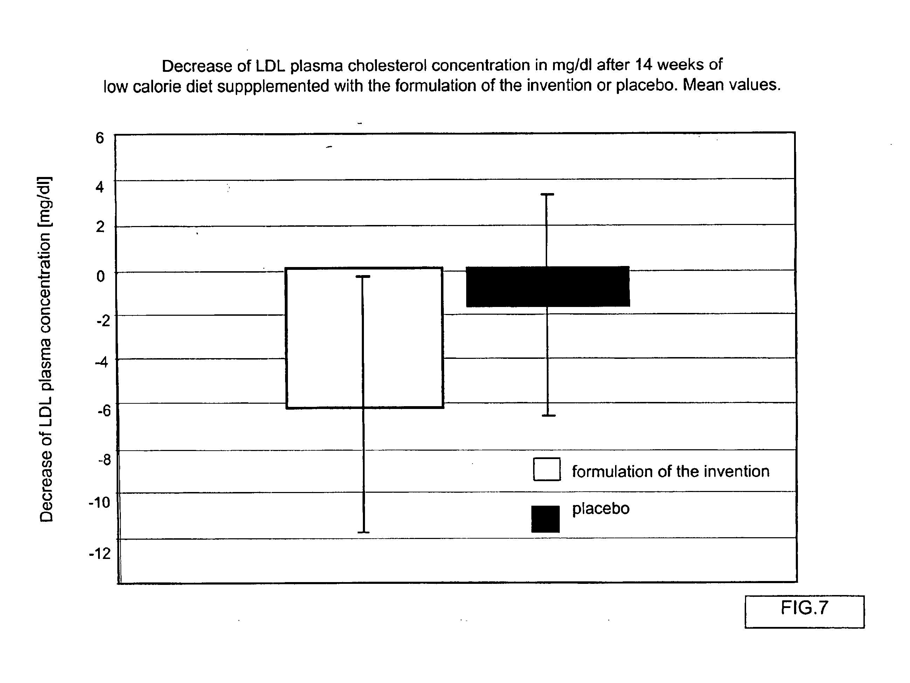

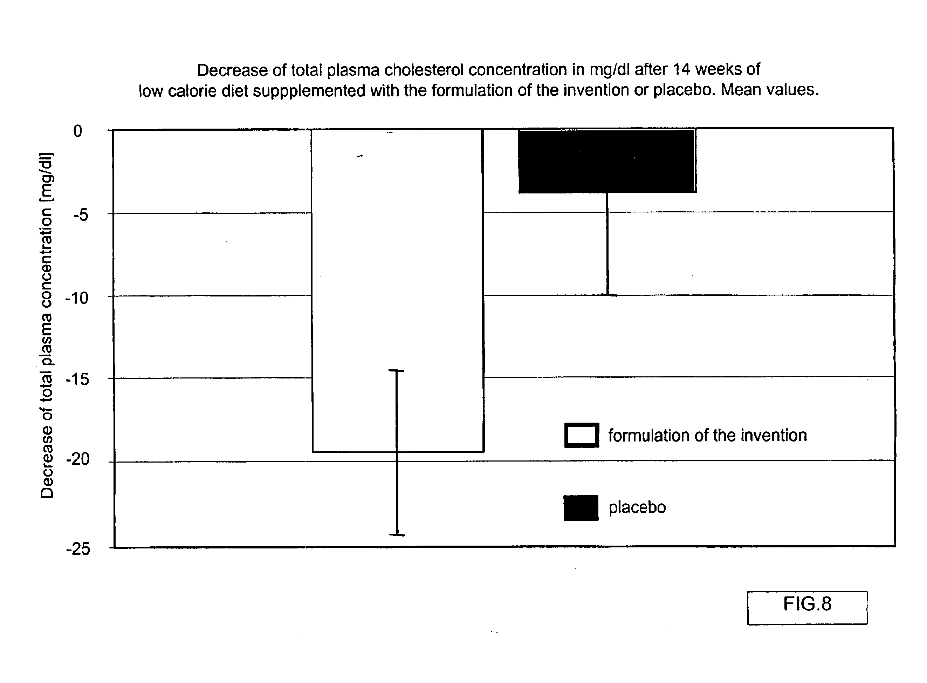

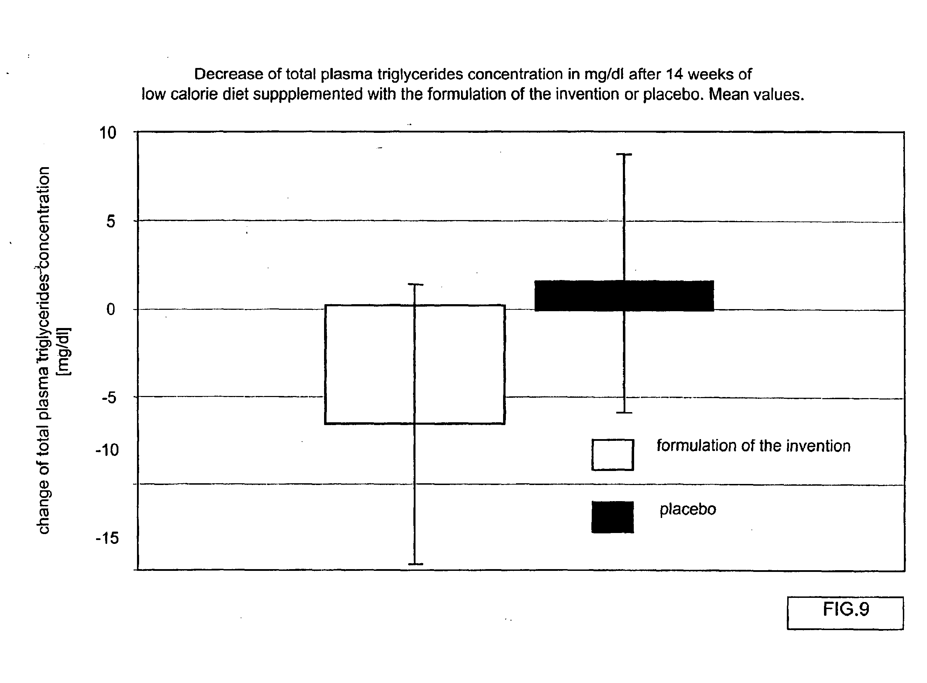

Decrease of body fat concentration was equal to -4.0 kg in the control group and -6.6 kg in the treated group (fig 6). Simultaneously, decrease of LDL plasma cholesterol concentration by 1.58 mg/dl in the control group and by 6.08 mg/dl in the treated group (fig.7) and total plasma cholesterol by 3.92 mg/dl and 19.42 mg/dl (fig.8) respectively was observed. Reduction of cholesterol concentration was accompanied by decrease of plasma triglyceride concentration by 6.63 mg/dl in the treated group, while in the control group triglyceride concentration increased by 1.42 mg/dl (fig.9).

The quoted studies have verified
a) Formulation of the invention is accelerating weight loss during dietary treatment of obesity.
b) Formulation of the invention contains active components which effects are synergistically strengthening each other, so that effect of their mixture is significantly greater them the effect of any separate component alone.

## Claims

1. Formulation for treating obesity and associated metabolic syndrome, comprising a combination of vegetable extracts, **characterized in that** it consists of:
a) 20-90% wt. of Green tea extract, containing more than 70 % of catechines, preferably containing Epigallocatehin galate (EGCG),
b) 2-30 % wt. of *Coleus forskholii* extract, containing at least 10 % of diterpene forskolin,
c) 5-58 % wt. of Yerba Maté extract, containing 2-4 % of caffeine and caffeoylquinic acids (CGA),
d) 7.5-45 wt. of *Betula alba* extract containing at most 3% of flavonides.

2. Formulation according to claim 1, **characterized in that** it further comprises an effective amount of vegetable extract of white kidney beans (*Phaseolus Vulgaris*).

3. Formulation according to claim 1 or 2, **characterized in that** Green tea extract is an extract obtained by water and/or ethyl acetate and water extraction in low temperature under reduced pressure.

4. Formulation according to claim 1 or 2, **characterized in that** Green tea extract is an extract obtained by alcohol extraction or extraction conducted in the presence of fat solvents for example selected from a group consisting of: methanol-chloroform mixture, alcohol ethers and detergents, in low temperature under reduced pressure.

5. Formulation according to claim 3 or 4, **characterized in that** Green tea extract comprises at least 30 % of EGCG

6. Formulation according to claim 3 or 4, **characterized in that** Green tea extract comprises at least 50 % of EGCG

7. Formulation according to claim 3 or 4, **characterized in that** Green tea extract comprises at least 80 % of EGCG

8. Formulation according to any of the claims 1-7, **characterized in that** it further comprises non-active exicipients or fillers selected from a group consisting of: silicon dioxide, magnesium stearate, laurylsulphate, other surfactants for example selected from a group consisting of: sodium carboxymethylcellulose, hydroxypropylmethyl cellulose and microcrystalline cellulose, anty-caking agents such as dicalcium phosphate; and materials forming the shell of the capsule.

9. Formulation for treating obesity and associated metabolic syndrome, comprising a combination of selected vegetable extracts, **characterized in that** it consists of:
a) 20-80% wt. of Green tea extract, containing more than 70 % of catechines, preferably containing Epigallocatehin galate (EGCG),
b) 2-30 % wt. of *Coleus forskholii* extract, containing at least 10 % of diterpene forskolin.
c) 5-50 % wt. of Guarana extract, containing more than 8 % of caffeine and caffeine-like polyphenoles (chlorogenic acids - CGA)
d) 7.5-45 % wt. of *Betula alba* extract containing at most 3% of flavonides.

10. Formulation according to claim 9, **characterized in that** it further comprises an effective amount of vegetable extract of white kidney beans (*Phaseolus Vulgaris*).

11. Formulation according to claim 9 or 10, **characterized in that** Green tea extract is an extract obtained by water and/or ethyl acetate and water extraction in low temperature under reduced pressure.

12. Formulation according to claim 9 or 10, **characterized in that** Green tea extract is an extract obtained by alcohol extraction or extraction conducted in the presence of fat solvents for example selected from a group consisting of: methanol-chloroform mixture, alcohol ethers and detergents, in low temperature under reduced pressure.

13. Formulation according to claim 11 or 12, **characterized in that** Green tea extract comprises at least 30 % of EGCG

14. Formulation according to claim 11 or 12, **characterized in that** Green tea extract comprises at least 50 % of EGCG

15. Formulation according to claim 11 or 12, **characterized in that** Green tea extract comprises at least 80 % of EGCG

16. Formulation according to any of the claims 9-15, **characterized in that** it further comprises non-active exicipients or fillers selected from a group consisting of silicon dioxide, magnesium stearate, laurylsulphate, other surfactants for example selected from a group consisting of: sodium carboxymethylcellulose, hydroxypropylmethyl cellulose and microcrystalline cellulose, anty-caking agents such as dicalcium phosphate; and materials forming the shell of the capsule.

## Patentansprüche

1. Formulierung zur Behandlung von Fettleibigkeit und assoziiertem Stoffwechselsyndrom, mit einer Kombination von Gemüseextrakten, **dadurch gekennzeichnet, dass** sie besteht aus:
a) 20 bis 90 Gewichtsprozent Extrakt von Grünem Tee, mit mehr als 70 % Katechinen, vorzugsweise enthalten Epicallocatehingallat (EGCG),
b) 2 - 30 Gewichtsprozent Extrakt von *Coleus Forskholii*, mit wenigstens 10 % Diterpenforskolin,
c) 5 - 58 Gewichtsprozent Extrakt von Yerba Mate, mit 2 - 4 % Koffein und Kaffeeoylquiniksäuren (CGA),
d) 7,5 - 45 Gewichtsprozent Extrakt von *Betula Alba,* mit höchstens 3 % Flavoniden.

2. Formulierung nach Anspruch 1, **dadurch gekennzeichnet, dass** sie außerdem eine wirksame Menge eines Gemüseextrakts von weißen Bohnen (*Phaseolus Vulgaris*) enthält.

3. Formulierung nach Anspruch 1 oder 2, **dadurch gekennzeichnet, dass** das Extrakt von Grünem Tee ein Extrakt ist, welches durch Wasser und/oder Ethylacetat und eine Wasserextraktion bei niedriger Temperatur unter vermindertem Druck erhalten wird.

4. Formulierung nach Anspruch 1 oder 2, **dadurch gekennzeichnet, dass** das Extrakt von Grünem Tee ein Extrakt ist, welches durch Alkoholextraktion oder durch eine Extraktion, welche in Gegenwart von Fettlösern durchgeführt wird, welche beispielsweise ausgewählt sind aus einer Gruppe, welche aus einer Methanol-Chloroform-Mischung, Alkoholethern und Detergentien besteht, bei niedriger Temperatur unter vermindertem Druck, erhalten wird.

5. Formulierung nach Anspruch 3 oder 4, **dadurch gekennzeichnet, dass** das Extrakt von Grünem Tee wenigstens 30 % EGCG enthält.

6. Formulierung nach Anspruch 3 oder 4, **dadurch gekennzeichnet, dass** das Extrakt von Grünem Tee wenigstens 50 % EGCG enthält.

7. Formulierung nach Anspruch 3 oder 4, **dadurch gekennzeichnet, dass** das Extrakt von Grünem Tee wenigstens 80 % EGCG enthält.

8. Formulierung nach einem der Ansprüche 1 bis 7, dadurch, dass sie außerdem nicht aktive Exicipienten oder Füllstoffe aufweist, welche aus einer Gruppe ausgewählt sind, welche besteht aus: Silikondioxid, Magnesiumstearat, Laurylsulphat, anderen oberflächenaktiven Stoffen, beispielsweise ausgewählt aus einer Gruppe, welche besteht aus: Natriumcarboxymethylzellulose, Hydroxypropylmethylzellulose und Mikrokristallinazellulose, Antiverbackungswirkstoffe, wie zum Beispiel Dikalciumphosphat und Materialien, welche die Ummantelung der Kapsel bilden.

9. Formulierung zur Behandlung von Fettleibigkeit und assoziiertem Stoffwechselsyndrom, mit einer Kombination von Gemüseextrakten, **dadurch gekennzeichnet, dass** sie besteht aus:
a) 20 bis 80 Gewichtsprozent Extrakt von Grünem Tee, mit mehr als 70 % Katechinen, vorzugsweise enthalten Epicallocatehingallat (EGCG),
b) 2 - 30 Gewichtsprozent Extrakt von *Coleus Forskholii,* mit wenigstens 10 % Diterpenforskolin,
c) 5 - 50 Gewichtsprozent Extrakt von Yerba Mate, mit 2 - 4 % Koffein und Kaffeeoylquiniksäuren (CGA),
d) 7,5 - 45 Gewichtsprozent Extrakt von *Betula Alba*, mit höchstens 3 % Flavoniden.

10. Formulierung nach Anspruch 9, **dadurch gekennzeichnet, dass** sie außerdem eine wirksame Menge eines Gemüseextrakts von weißen Bohnen (Phaseolus Vulgaris) enthält.

11. Formulierung nach Anspruch 9 oder 10, **dadurch gekennzeichnet, dass** das Extrakt von Grünem Tee ein Extrakt ist, welches durch Wasser und/oder Ethylacetat und eine Wasserextraktion bei niedriger Temperatur unter vermindertem Druck erhalten wird.

12. Formulierung nach Anspruch 9 oder 10, **dadurch gekennzeichnet, dass** das Extrakt von Grünem Tee ein Extrakt ist, welches durch Alkoholextraktion oder durch eine Extraktion, welche in Gegenwart von Fettlösern durchgeführt wird, welche beispielsweise ausgewählt sind aus einer Gruppe, welche aus einer Methanol-Chloroform-Mischung, Alkoholethern und Detergentien besteht, bei niedriger Temperatur unter vermindertem Druck, erhalten wird.

13. Formulierung nach Anspruch 11 oder 12, **dadurch gekennzeichnet, dass** das Extrakt von Grünem Tee wenigstens 30 % EGCG enthält.

14. Formulierung nach Anspruch 11 oder 12, **dadurch gekennzeichnet, dass** das Extrakt von Grünem Tee wenigstens 50 % EGCG enthält.

15. Formulierung nach Anspruch 11 oder 12, **dadurch gekennzeichnet, dass** das Extrakt von Grünem Tee wenigstens 80 % EGCG enthält.

16. Formulierung nach einem der Ansprüche 9 bis 15, dadurch, dass sie außerdem nicht aktive Exicipienten oder Füllstoffe aufweist, welche aus einer Gruppe ausgewählt sind, welche besteht aus: Silikondioxid, Magnesiumstearat, Laurylsulphat, anderen oberflächenaktiven Stoffen, beispielsweise ausgewählt aus einer Gruppe, welche besteht aus Natriumcarboxymethylzellulose, Hydroxypropylmethylzellulose und Mikrokristallinazellulose, Antiverbackungswirkstoffe, wie zum Beispiel Dikalciumphosphat und Materialien, welche die Ummantelung der Kapsel bilden.

## Revendications

1. Formulation pour le traitement de l'obésité et du syndrome métabolique associé, comprenant une association d'extraits végétaux,
**caractérisée en ce qu'**
elle est constituée de :
a) 20 % à 90 % P/P d'extrait de thé vert, contenant plus de 70 % de catéchines, contenant de préférence de l'épigallocatéchine gallate (EGCG),
b) 2 % à 30 % P/P d'extrait de *Coleus forskholii*, contenant au moins 10 % de diterpène forskoline,
c) 5 % à 58 % P/P d'extrait de maté, contenant 2 % à 4 % de caféine et d'acides caffeoylquiniques (CGA),
d) 7,5 % à 45 % P/P d'extrait de *Betula alba*, contenant au moins 3 % de flavonoïdes.

2. Formulation selon la revendication 1,
**caractérisée en ce qu'**
elle comprend également une quantité efficace d'extrait végétal de haricot blanc *(Phaseolus vulgaris).*

3. Formulation selon la revendication 1 ou 2,
**caractérisée en ce que**
l'extrait de thé vert est un extrait obtenu par extraction à l'eau et/ou à l'acétate d'éthyle et à l'eau à basse température sous pression réduite.

4. Formulation selon la revendication 1 ou 2,
**caractérisée en ce que**
l'extrait de thé vert est un extrait obtenu par extraction alcoolique ou extraction effectuée en présence de solvants gras par exemple sélectionnés dans un groupe composé de : mélange méthanol et chloroforme, éthers d'alcool et détergents, à basse température sous pression réduite.

5. Formulation selon la revendication 3 ou 4,
**caractérisée en ce que**
l'extrait de thé vert comprend au moins 30 % d'EGCG.

6. Formulation selon la revendication 3 ou 4,
**caractérisée en ce que**
l'extrait de thé vert comprend au moins 50 % d'EGCG.

7. Formulation selon la revendication 3 ou 4,
**caractérisée en ce que**
l'extrait de thé vert comprend au moins 80 % d'EGCG.

8. Formulation selon l'une des revendications 1 à 7,
**caractérisée en ce qu'**
elle comprend également des excipients non actifs ou des agents de remplissage sélectionnés dans un groupe composé de : dioxyde de silicone, stéarate de magnésium, laurylsulfate, d'autres surfactants par exemple sélectionnés dans un groupe composé de : carboxyméthylcellulose sodique, hydroxypropylméthyl cellulose et cellulose microcristalline, des agents antiagglomérants tels que phosphate dicalcique ; et des matériaux formant l'enveloppe de la gélule.

9. Formulation pour le traitement de l'obésité et du syndrome métabolique associé, comprenant une association d'extraits végétaux,
**caractérisée en ce qu'** elle est constituée de :
a) 20 % à 80 % P/P d'extrait de thé vert, contenant plus de 70 % de catéchines, contenant de préférence de l'épigallocatéchine gallate (EGCG),
b) 2 % à 30 % P/P d'extrait de *Coleus forskholii*, contenant au moins 10 % de diterpène forskoline,
c) 5 % à 50 % P/P d'extrait de guarana, contenant plus de 8 % de caféine et de polyphénols de type caféine (acides chlorogéniques - CGA),
d) 7,5 % à 45 % P/P d'extrait de *Betula alba*, contenant au moins 3 % de flavonoïdes.

10. Formulation selon la revendication 9,
**caractérisée en ce qu'**
elle contient également une quantité efficace d'extrait végétal de haricot blanc *(Phaseolus vulgaris).*

11. Formulation selon la revendication 9 ou 10,
**caractérisée en ce que**
l'extrait de thé vert est un extrait obtenu par extraction à l'eau et/ou à l'acétate d'éthyle et à l'eau à basse température sous pression réduite.

12. Formulation selon la revendication 9 ou 10,
**caractérisée en ce que**
l'extrait de thé vert est un extrait obtenu par extraction alcoolique ou extraction effectuée en présence de solvants gras par exemple sélectionnés dans un groupe composé de : mélange méthanol et chloroforme, éthers d'alcool et détergents, à basse température sous pression réduite.

13. Formulation selon la revendication 11 ou 12,
**caractérisée en ce que**
l'extrait de thé vert comprend au moins 30 % d'EGCG.

14. Formulation selon la revendication 11 ou 12,
**caractérisée en ce que**
l'extrait de thé vert comprend au moins 50 % d'EGCG.

15. Formulation selon la revendication 11 ou 12,
**caractérisée en ce que**
l'extrait de thé vert comprend au moins 80 % d'EGCG.

16. Formulation selon l'une des revendications 9 à 15,
**caractérisée en ce qu'**
elle comprend également des excipients non actifs ou des agents de remplissage sélectionnés dans un groupe composé de dioxyde de silicone, stéarate de magnésium, laurylsulfate, d'autres surfactants par exemple sélectionnés dans un groupe composé de : carboxyméthylcellulose sodique, hydroxypropylméthyl cellulose et cellulose microcristalline, des agents antiagglomérants tels que phosphate dicalcique ; et des matériaux formant l'enveloppe de la gélule.
